# EUROPÄISCHE PATENTSCHRIFT

(11) **EP 1 532 129 B1**
(45) Veröffentlichungstag und Bekanntmachung des Hinweises auf die Patenterteilung: **22.11.2006**
(21) Anmeldenummer: 03797201.5
(22) Anmeldetag: 17.07.2003
(51) Int. Cl.: C07D 307/86, A61K 31/343, A61P 31/12

(54) **SORBICILLACTON-A-DERIVATE ZUR BEHANDLUNG VON TUMOR- UND VIRUSERKRANKUNGEN**
SORBICILLACTONE-A DERIVATIVES FOR THE TREATMENT OF TUMOUR AND VIRAL DISEASES
DERIVES DE SORBICILLACTONE-A DESTINES AU TRAITEMENT DE MALADIES TUMORALES ET VIRALES

(30) Priorität: 21.08.2002 DE 10238257
(43) Veröffentlichungstag der Anmeldung: 25.05.2005
(73) Patentinhaber: Johannes Gutenberg-Universität Mainz, 55122 Mainz (DE); Freistaat Bayern vertreten durch die Julius-Maximilians-Universität Würzburg, 97070 Würzburg (DE)
(72) Erfinder: BRINGMANN, Gerhard, 97074 Würzburg (DE); LANG, Gerhard, 97072 Würzburg (DE); MÜHLBACHER, Jörg, 79106 Freiburg (DE); SCHAUMANN, Karsten, 27578 Bremerhaven (DE); STEFFENS, Stefan, 52072 Aachen (DE); MÜLLER, Werner, E., G., 65203 Wiesbaden (DE)
(74) Vertreter: Krauss, Jan
(86) Internationale Anmeldenummer: PCT/EP2003/007805
(87) Internationale Veröffentlichungsnummer: WO 2004/026854

(56) Entgegenhaltungen:
- WO-A-02/26726
- US-A- 5 925 636

## Beschreibung

Die vorliegende Erfindung betrifft neue bioaktive Verbindungen aus marinen Organismen, die als Sorbicillacton A bezeichnet werden und deren Derivate. Die Erfindung betrifft weiterhin ein Verfahren zur Herstellung der Verbindungen, diese enthaltende Arzneimittel und deren Verwendung bei der Behandlung von Erkrankungen. Darüber hinaus wird eine Synthese von Sorbicillacton A und deren Derivaten beschrieben.

### Hintergrund der Erfindung

Marine eukaryontischen Organismen, speziell Schwämme, Hydrozoen, Bryozoen und Tunikaten, stellen eine sehr reiche Quelle für bioaktive Substanzen dar (Sarma AS, Daum T, Müller WEG (1993) Secondary metabolites from marine sponges. Akademie gemeinnütziger Wissenschaften zu Erfurt, Ullstein-Mosby Verlag, Berlin). Grund hierfür ist die Tatsache, daß diese Vielzeller sessile Organismen sind, die sich aus dem im Umgebungsmilieu vorhandenen Mikroorganismen ernähren. Hierfür brauchen sie effiziente Abwehrmechanismen, um sich vor Bakterien- und Pilzinfektionen zu schützen. Die aktivsten Abwehrsubstanzen stellen solche sekundäre Metabolite dar, die von den Symbionten gebildet werden, die in den marinen eukaryontischen Organismen vorkommen. Deshalb bleibt es in den meisten Fällen bis heute unklar, ob der Wirt (Schwämme, Hydrozoen, Bryozoen und Tunikaten) oder die mit ihnen häufig in eine Symbiose lebenden Mikroorganismen (Pilze, Bakterien) die Produzenten für die bioaktiven Substanzen sind (Althoff et al. (1998) Marine Biol 130:529-536; Wiens et al., Marine Biol.; im Druck). In den letzten Jahren hat sich auch erwiesen, daß Schwämme Abwehrmechanismen besitzen, um Viren zu eliminieren (Grebenjuk et al. (2002) Europ. J. Biochem. 269: 1382-1392). Folglich ist es ein Hauptziel in den Forschungen, diese Mikroorganismen in Kultur zu züchten und sie dort ihre bioaktiven Substanzen produzieren zu lassen.

Es kann heute angenommen werden, daß weniger als 5% der in marinen eukaryontischen Organismen vorkommende Bakterien und Pilze kultivierbar sind. Folglich ist auch deren Potential noch nicht genutzt. Zur erfolgreichen nachhaltigen Nutzung des bioaktiven Potentials ist es deshalb notwendig, optimale Kultivierungsmethoden für Mikroorganismen zu entwickeln, mit hoher Ausbeute die bioaktiven Substanzen aus dem Medium zu gewinnen, um diese mit effizienten Reinigungs- und Charakterisierungsverfahren zu identifizieren. Bis heute ist nur ein Medikament in die klinische Therapie eingeführt, das von marinen eukaryontischen Organismen produziert wird, das 9-β-D-Arabinofuranosyladenosin [araA] (Müller et al. (1977) Ann. New York Acad Sci 284:34-48).

Das Anwendungsspektrum für eine kommerzielle Nutzung der bioaktiven Sekundärmetabolite ist breit und reicht von einer Behandlung von Tumoren, von neurodegenerativen Erkrankungen bis zur Therapie von Infektionen hervorgerufen von Bakterien, Viren und/oder Pilzen.

Intensiv wird nach solchen bioaktiven Substanzen gefahndet, die eine hohe Spezifität gegen definierte Tumoren entfalten und gleichzeitig opportunistische Infektionen, z.B. durch Viren, eindämmen.

Es ist daher Aufgabe der vorliegenden Erfindung, eine neue bioaktive Substanz aus einem marinen Organismus, ein Verfahren zu dessen Herstellung und dessen Verwendungen zur Verfügung zu stellen.

Erfindungsgemäß wird diese Aufgabe zunächst durch das zur Verfügung stellen als Sorbicillacton A und Sorbicillacton-A-Derivate bezeichneter bioaktiver Substanzen gelöst. Sorbicillacton A ist ein Naturstoff, der bisher noch nicht beschrieben wurde. Deshalb ist auch sein Wirkspektrum noch weitgehend unbekannt. In der Literatur sind auch keine zu Sorbicillacton A verwandten Substanzen beschrieben worden.

Es wurde weiterhin gefunden, daß Sorbicillacton A und die abgeleiteten Sorbicillacton-A-Derivate der allgemeinen Formeln (1) und (2) ausgeprägte antitumorale und antivirale Eigenschaften besitzen. Erfindungsgemäß werden somit Verbindungen der allgemeinen Formel (2): 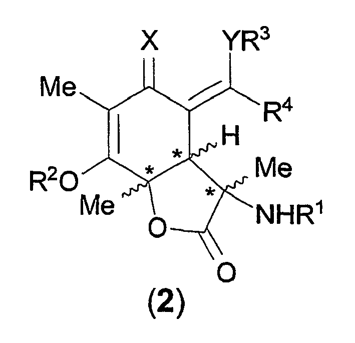 worin
R¹ ist ausgewählt aus: -H, (C₁-C₁₀)-Alkyl, wobei Alkyl gerade oder verzweigt ist, (C₃₋C₁₀)-Alkenyl oder eine Acylgruppe (z.B. Formyl, Acetyl, Trichloracetyl, Fumaryl, Maleyl, Succinyl usw.), wobei eventuelle freie -COOH-Gruppen an dieser Acylgruppe auch in Form von Estern (z.B. eines Methylesters, -COOMe) vorliegen können oder R¹ gegebenenfalls auch einer der beiden heterocyclischen Acylsubstituenten **(3)** oder **(4)** sein kann 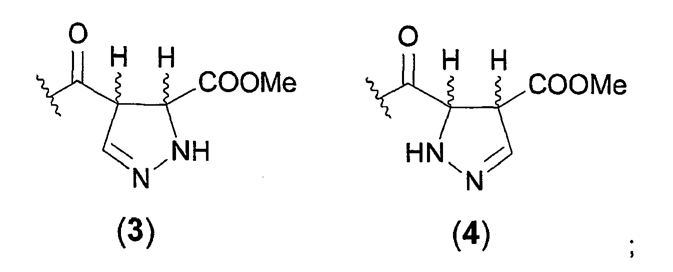
R² ausgewählt ist aus: -H, (C₁-C₁₀)-Alkyl, wobei Alkyl gerade oder verzweigt ist oder einer Acylgruppe (z.B. Formyl, Acetyl usw.);
R³ ausgewählt ist aus: -H, (C₁-C₁₀)-Alkyl, wobei Alkyl gerade oder verzweigt ist oder eine Acylgruppe (z.B. Formyl, Acetyl usw.);
R⁴ ausgewählt ist aus: (C₁-C₁₀)-Alkyl, wobei Alkyl gerade oder verzweigt ist oder (C₃₋C₁₀)-Alkenyl, wobei der Alkenylrest entweder eine oder mehrere Doppelbindungen enthalten kann;
X ausgewählt ist aus O, S, NOH oder NOR⁵, wobei R⁵ ein geradekettiges oder verzweigtes (C₁-C₆)-Alkyl ist;
Y entweder O ist oder Y und X sind zwei miteinander verbundene N-Atome, die somit einen Pyrazolring bilden.
und wobei die Verbindung als *(R,R,R)-, (R,R,S)-, (R,S,R)-, (R,S,S)-, (S,R,R)-, (S,R,S)-, (S,S,R)-* und *(S,S,S)*-Stereoisomer vorliegen kann und pharmazeutisch verträgliche Salze oder Solvate von (2) zur Verfügung gestellt.

Erfindungsgemäß bevorzugt ist dabei eine Verbindung mit der Formel (1):

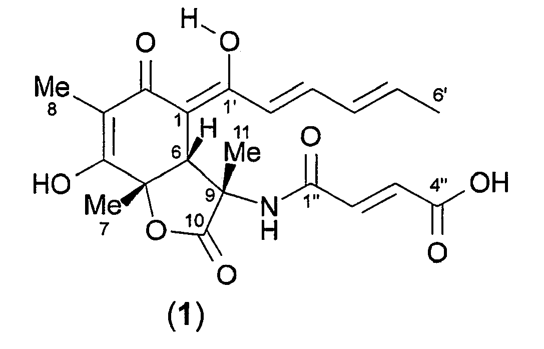

(Sorbicillacton A) oder Derivate davon, deren Diastereomere, sowie die entsprechenden Enantiomere und pharmazeutisch verträgliche Salze oder Solvate dieser Verbindung.

Im Rahmen der vorliegenden Erfindung soll unter einem "Derivat" eine von der allgemeinen Formel (2) abgeleitete Verbindung sein, die zum Beispiel durch verschiedene der oben für R₁ bis R₄ und X oder Y angegebenen Restgruppen substituiert ist, sowie Gemische verschiedener dieser Verbindungen, die zum Beispiel zu einem auf die jeweils zu therapierende Erkrankung und/oder den Patienten auf Basis von diagnostischen oder Daten über den Behandlungserfolg oder -verlauf abgestimmten "personalisierten" Medikament verarbeitet werden können. Unter einem Derivat wird auch eine Verbindung der Sorbicillacton A-Klasse verstanden, die aus anderen (z.B.) marinen Organismen isoliert werden kann, als den hier (beispielhaft) erwähnten.

Unter einem "Vorläufer" einer Substanz soll im Rahmen der vorliegenden Erfindung zum einen eine Substanz verstanden werden, die im Laufe ihrer Verabreichung zur Behandlung durch die Bedingungen im Körper (z.B. pH im Magen, oder ähnliches) so verändert wird, oder aber durch den Körper nach Aufnahme so metabolisiert wird, daß sich als wirksame Substanz die erfindungsgemäße Verbindung oder deren Derivate bilden. Zum anderen werden als Vorläufer aus Organismen isolierte Derivate von Sorbicillacton A verstanden, die als Ausgangsprodukte zur Synthese der Verbindung in dem jeweiligen Organismus dienen und bereits die hierin angegebenen Eigenschaften des Sorbicillacton A aufweisen.

Es wurde nun gefunden, daß Sorbicillacton A und die von dieser Substanz abgeleiteten Sorbicillacton-A-Derivate eine ausgeprägte und nicht vorhersehbare antitumorale und antivirale Eigenschaft besitzen. Weiterhin wurden überraschenderweise entzündungshemmende Eigenschaften der neuen Substanz gefunden. Aufgrund dieser Eigenschaften und basierend auf dem Befund, daß Sorbicillacton A und die Sorbicillacton-A-Derivate weitgehend nicht toxisch für Säugetiere (Beispiel Maus) sind, sind die hier beschriebenen Substanzen geeignet zur Behandlung von Tumoren und Viruserkrankungen. Es wird empfohlen, diese Substanzen entweder in der vorliegenden Form oder in Form einer Depotsubstanz oder als Vorläufer zusammen mit einer geeigneten, pharmazeutisch verträglichen Verdünnungslösung oder Trägersubstanz anzuwenden.

Aus marinen Quellen, Invertebraten und auch Mikroorganismen, ist bereits eine Vielzahl von Naturstoffen beschrieben. Für die Suche nach neuen biologisch aktiven Verbindungen ist daher eine effiziente Dereplikation nötig, das heißt, bekannte Stoffe sollten bereits in einer frühen Phase des Arbeitsprozesses aufgefunden und eindeutig identifiziert werden. Zu diesem Zweck eignet sich besonders die Hochleistungsflüssigchromatographie (HPLC) in Kopplung mit verschiedenen spektroskopischen Detektionsmethoden. Besonders leistungsfähig ist hier die ,LC-Triade', eine Kopplung von HPLC mit NMR, MS, und CD (Bringmann et al. (1998) Anal. Chem. 70:2805-2811; Bringmann et al. (1999) Anal. Chem. 71:2678-2686). Die damit erhaltenen spektroskopischen Daten von Extraktinhaltsstoffen, ohne eigentliche Isolierung der einzelnen Verbindungen, ermöglichen durch Suche in Datenbanken die eindeutige Identifizierung schon bekannter Substanzen. Darüber hinaus ist unter geeigneten Umständen sogar die Aufklärung der vollständigen Stereostruktur unbekannter Naturstoffe, ohne vorherige Isolierung, möglich.

Sorbicillacton A ist ein Naturstoff und Sorbicillacton-A-Derivate sind davon abgeleitete Syntheseprodukte, die bisher unbekannt waren und deren Wirkung nicht beschrieben ist.

Diese erfindungsgemäßen Verbindungen können mit den üblichen Lösungsmitteln, Hilfs- und Trägerstoffen zu Tabletten, Dragees, Kapseln, Tropflösungen, Suppositorien, Injektions- und Infusionszubereitungen verarbeitet werden, um therapeutisch zur peroralen, rektalen oder parenteralen Applikation Verwendung zu finden.

Die vorliegende Erfindung betrifft weiterhin ein Verfahren zur Herstellung einer oben genannten Verbindung, das dadurch gekennzeichnet ist, daß die Substanz bevorzugt aus einem marinen Organismus, wie zum Beispiel einem in einem marinen Schwamm als Symbiont wachsenden Pilz der Gattung *Penicillium* isoliert wird. Im Rahmen der vorliegenden Erfindung wurde ein Pilz der Gattung *Penicillium* (vorzugsweise *Penicillium chrysogenum*) erstmalig als Produzent der erfindungsgemäßen Verbindungen nachgewiesen. Sorbicillacton A wird unter den nachstehend aufgeführten Kulturbedingungen sowohl in das Kulturmedium abgegeben, als auch vor allem in der Pilzbiomasse akkumuliert. Bei dem Pilz handelt es sich um einen Vertreter der Gattung *Penicillium* LINK (1809), die als anamorphe Trichocomacae / Deuteromycetes / Mitosporic Fungi, code group 1.A2.15 systematisch charakterisiert ist. Diese Gattung zeichnet sich durch eine Vielzahl weit verbreiteter Spezies aus, die zum Teil beachtliche biochemische Potenzen besitzen. Bei der vorliegenden Spezies handelt es sich um eine seit 1910 bekannte Art, die bisher als Bewohner terrestrischer Biotope beschrieben ist. Die vorliegende Pilzisolation (Pilzstamm) entstammt jedoch dem marin-aquatischen Lebensraum und wurde von dem marinen Schwamm *Ircinia fasciculata* (Porifera) isoliert. Aufgrund der im Rahmen dieser Erfindung erfolgten Strukturaufklärung läßt sich die erfindungsgemäße Verbindung jedoch auch mittels herkömmlicher Synthesechemie herstellen, bzw. zu Derivaten und Vorläufern modifizieren. Bevorzugt ist dabei als weiterer Aspekt der vorliegenden Erfindung ein Verfahren zur biomimetischen Synthese einer erfindungsgemäßen Verbindung, bei dem zunächst Sorbicillin und/oder Derivaten davon zur Verfügung gestellt werden, dann auf an sich bekannte Weise eine oxidative Dearomatisierung und anschließende Addition von Alanin (im Falle von Sorbicillin A) oder anderen Aminosäuren und deren Analoga (für andere Derivate von Sorbicillin) durchgeführt wird, und eine anschließende Anknüfung von Fumarsäure (im Falle von Sorbicillin A) oder analogen Acylresten (für andere Derivate von Sorbicillin) erfolgt.

Ein weiterer Aspekt der vorliegenden Erfindung betrifft die Verwendung mindestens einer der oben genannten Verbindungen zur Behandlung von Erkrankungen, wie z.B. Tumor- und/oder Viruserkrankungen und/oder zur Behandlung von Entzündungszuständen. Diese Verwendung kann zum Beispiel in Form einer Depotsubstanz oder als Vorläufer zusammen mit einer geeigneten, pharmazeutisch verträglichen Verdünnungslösung oder Trägersubstanz erfolgen. Im Falle der Behandlung von HIV-1 assoziierten Erkrankungen ist eine Behandlung in einem Konzentrationsbereich zwischen 0,3 und 3,0 µg/ml bevorzugt, im Falle der Behandlung von Entzündungen eine Konzentration von ungefähr 2 µg/ml. Bei der Behandlung von Ödembildung wird normalerweise eine Menge von ungefähr 20 µg der oben genannten Verbindung verwendet. Ein weiterer Aspekt ist die Verwendung einer oder mehrerer der erfindungsgemäßen Verbindung(en) Herstellung eines Medikaments zur Behandlung von Tumor- und/oder Viruserkrankungen und/oder zur Behandlung von Entzündungszuständen. Diese Herstellung kann analog zu der oben beschriebenen Weise erfolgen und in den oben beschriebenen Konzentrationen und für die oben u.a. beschriebenen Anwendungen erfolgen.

Ein weiterer Aspekt der vorliegenden Erfindung betrifft eine pharmazeutische Zusammensetzung, umfassend eine erfindungsgemäße Verbindung, zusammen mit geeigneten Zusatz- oder Hilfsstoffen. Diese pharmazeutische Zusammensetzung kann dadurch gekennzeichnet sein, daß die Verbindung in Form einer Depotsubstanz oder als Vorläufer zusammen mit einer geeigneten, pharmazeutisch verträglichen Verdünnungslösung oder Trägersubstanz vorliegt.

Besonders bevorzugt sind pharmazeutische Zusammensetzung, in denen die erfindungsgemäße Verbindung in einer Menge von 20 µg vorliegt (besonders geeignet zur Behandlung von Ödembildung) oder pharmazeutische Zusammensetzungen, in denen die erfindungsgemäße Verbindung in solch einer Menge vorliegt, daß ein Konzentrationsbereich zwischen 0,3 und 3,0 µg/ml bei der Behandlung in vivo vorliegt (besonders geeignet zur Behandlung von viralen und/oder Entzündungserkrankung).

Bevorzugt ist eine pharmazeutische Zusammensetzung gemäß der vorliegenden Erfindung, die weitere Chemotherapeutika enthält. Diese Chemotherapeutika können alle für den Fachmann üblichen Chemotherapeutika im Rahmen einer Krebstherapie umfassen (z.B. Taxol oder andere).

Erfindungsgemäß kann die oben genannte pharmazeutische Zusammensetzung in Form von Tabletten, Dragees, Kapseln, Tropflösungen, Suppositorien, Injektions- oder Infusionszubereitungen zur peroralen, rektalen oder parenteralen Verwendung vorliegen. Solche Darreichungsformen und deren Herstellung sind dem Fachmann bekannt.

Die Verfahrensprodukte der allgemeinen Formeln **(1)** und **(2)** besitzen wertvolle pharmakologische Eigenschaften. Die Antitumorwirkung wurde u.a. am L5178y-Mäuselymphomzellsystem nachgewiesen (ATCC CRL 1722). Diese Zellen wurden in Suspensionskultur gehalten, wie bereits früher beschrieben (Müller et al. (1979) Cancer Res. 39: 1102-1107). Die ED₅₀-Konzentrationen für Sorbicillacton A (Inokulation: 10.000 Zellen/ml; Inkubationszeit: 72 Std.) lag bei diesen Tumor-Zellstämmen bei 2.2 ± 0.3 µg/ml. Etwas geringer wirksam war Sorbicillacton A bei den Tumorzell-Linien PC-12 (adrenaler, phaeochromocytomaler Tumor [Ratte]; ATCC CRL 1721), Sarcoma 180 (Mäuse-Sarkom; ATCC TIB 66) und HeLa S3 (epitheloides Carcinom [Cervix; Mensch]; ATCC CCL 2.2) mit ED₅₀-Konzentrationen zwischen 8 und 15 µg/ml.

Die vorliegende Erfindung beschreibt weiterhin ein Verfahren zur Behandlung einer Erkrankung, ausgewählt aus Tumor- und/oder Viruserkrankungen und/oder Entzündungszuständen, die das Verabreichen einer erfindungsgemäßen Verbindung, etwa in Form einer erfindungsgemäßen pharmazeutischen Zusammensetzung, umfaßt. Die Verabreichung kann erfindungsgemäß in Form einer Depotsubstanz oder als Vorläufer zusammen mit einer geeigneten, pharmazeutisch verträglichen Verdünnungslösung oder Trägersubstanz erfolgen.

Insbesondere wird ein Verfahren zur Behandlung offenbart, bei dem die Viruserkrankung eine HIV-1-Infektion ist. Dabei kann die Verbindung in einem Konzentrationsbereich in vivo zwischen 0,3 und 3,0 µg/ml erfolgen. Dem Fachmann sind die für die Erreichung dieser Konzentrationen erforderlichen zu verabreichenden Mengen leicht ersichtlich, die u.a. von dem jeweiligen Patienten, der Erkrankung und der Bioverfügbarkeit der jeweils verwendeten Verbindung abhängen können. Als weitere zu therapierende virale Erkrankungen seien hier beispielhaft weitere HIV-Infektionen, Infektionen mit HCV (Hepatitis C-Virus), Herpes- und/oder RSV-Virus-Erkrankungen genannt.

Weiterhin wird ein Verfahren zur Behandlung einer Erkrankung beschrieben, wobei eine Entzündung behandelt wird. Dabei kann die Verbindung analog zur Verabreichung bei viralen Erkrankungen in einer Konzentration von 2 µg/ml in vivo verabreicht werden. Eine weitere mögliche Behandlung ist die Therapie einer Ödembildung. Dabei kann eine erfindungsgemäße Verbindung in einer Menge von 20 µg verabreicht werden.

Von besonderer Bedeutung für die Verwendung der Verfahrensprodukte zur Chemotherapie von Tumorerkrankungen als auch für die antivirale Therapie ist die Tatsache, daß eine zytostatische Wirksamkeit auf nicht-tumorale Zellen in Kultur bei therapeutisch anzuwendenden Konzentrationen fehlt. Dieser Schluß wurde aus Versuchen mit Lymphozyten-Kulturen gezogen: Milz-Lymphozyten wurden von sechs Wochen alten NMRI-Mäusen gewonnen; die Erythrozyten wurden aus der Suspension durch Ammoniumchlorid-Behandlung entfernt. Die Milz-Lymphozyten wurden in RPMI 1640-Medium mit 20% fötalem Kälberserum in einer Dichte von 1,5 x 10⁷ Zellen/ml für 72 Std. in Anwesenheit von 2 µg/ml Concanavalin A gehalten. 18 Std. vor Ende des Versuches wurde [³H]-Thymidin hinzu gegeben. Bei einer Inkubation mit 15 µg/ml der Verfahrensprodukte wurde keine Beeinträchtigung der DNA-Syntheserate gemessen. In Gegenwart von 20 µg/ml erfolgte eine lediglich 20%ige Hemmung der Einbaurate von [³H]-Thymidin in die DNA.

Die subakute Toxizität von Sorbicillacton A und seinen Derivaten liegt bei einer fünftägigen i.p.-Behandlung von Mäusen mit Werten bei >>20 mg/kg so günstig, daß eine Verwendung der Verfahrensprodukte bei einer Chemotherapie von Krebserkrankungen aussichtsreich ist.

Die ausgeprägte antivirale Wirksamkeit der Verfahrensprodukte wurde im HTLV-IIIB (HIV-1) Testsystem nachgewiesen. Eine spezifische Hemmung der Virus-Produktion wurde bei einem Konzentrationsbereich zwischen 0,3 und 3,0 µg/ml ermittelt.

Darüber hinaus entfalten Sorbicillacton A und Sorbicillacton-A-Derivate ausgeprägte entzündungshemmende Eigenschaften. Diese Effekte konnten sowohl *in vitro* (Modell: Hemmung der Phospholipase A2 [aus Bienengift]) als auch *in vivo* (Modell: Mäuse-Ohr Ödem) gemessen werden. In *In-vitro*-Versuchen mit Phospholipase A2 (aus Bienengift)konnte gezeigt werden, daß bei einer Konzentration von 2 µg/ml eine nahezu 80%-ige Hemmung erzielt werden. Weiterhin wurde der Effekt von Sorbicillacton-A auf das Mäuseödem gemessen. Das Ödem wurde bei den Mäusen (Swiss; etwa 25 g) mit TPA (10 µg) induziert. TPA wurde in Aceton gelöst und topisch auf die rechte innere Ohrmuschel aufgebracht. Die linke innere Ohrmuschel diente als Kontrolle (Aceton-Kontrolle). Durch cervikale Dislokalisation wurden die Tiere nach 4 Stunden getötet und die Ödembezirke ausgeschnitten. Diese wurden anschließend gewogen. Das Verhältnis zwischen dem Gewicht des behandelten Gewebes zu der Kontrolle diente als Maß für den Sorbicillacton-A-Effekt (Carlson RP et al. (1985) Agents Actions 17: 197-204). Die Wirksubstanz wurde gewöhnlich 10 min nach der TPA-Behandlung topisch auf die behandelte Stelle aufgebracht. Die Kontrollen entwickelten nach 3 Stunden ein Ödem von 12,3 ± 0,9 mg. Der Effekt von Sorbicillacton A auf die Ödembildung war signifikant (P < = 0.01); bei einer Behandlung mit 20 µg wurde eine 39.2 ± 5.3%-ige Hemmung erzielt (n = 5).

Die Erfindung soll nun im folgenden anhand von Beispielen näher verdeutlicht werden, ohne jedoch auf diese Beispiele irgendwie beschränkt zu sein.

### Beispiel 1: Gewinnung der Substanz Sorbicillacton A aus biologischen Material.

Ein Pilz der Gattung *Penicillium* (vorzugsweise *Penicillium chrysogenum*) wird erstmalig als Produzent des neuen Naturstoffes Sorbillacton A nachgewiesen. Sorbicillacton A wird unter den nachstehend aufgeführten Kulturbedingungen sowohl in das Kulturmedium abgegeben als auch vor allem in der Pilzbiomasse akkumuliert. Bei dem Pilz handelt es sich um einen Vertreter der Gattung *Penicillium* LINK (1809), die als anamorphe Trichocomacae / Deuteromycetes / Mitosporic Fungi, code group 1.A2.15 systematisch charakterisiert ist. Diese Gattung zeichnet sich durch eine Vielzahl weit verbreiteter Species aus, die zum Teil beachtliche biochemische Potenzen besitzen. Bei der vorliegenden Species handelt es sich um eine seit 1910 bekannte Art, die als Bewohner terrestrischer Biotope beschrieben ist. Die vorliegende Pilzisolation (Pilzstamm) entstammt jedoch dem marin-aquatischen Lebensraum und wurde von dem marinen Schwamm *Ircinia fasciculata* (Porifera) isoliert.

### Beschreibung des allgemeinen Verfahrens der Isolierung und Anzucht des Pilzes

Anschließend wird das gewachsene Mycel einschließlich der Kulturbrühe abgeerntet, mit 40 ml Ethylacetat pro 300 ml Kulturbrühe versetzt und bei -86°C tiefgefroren.

Zur Extraktion werden vorzugsweise Methanol, Dichlormethan und Essigester eingesetzt, es sind aber auch andere Lösungsmittel wie Ethanol, Propanol, Butanol, Ether, n-Hexan, Benzin, Toluol, Aceton, Methylethylketon, Essigsäure-Tertiärbutylester denkbar. Die erhaltenen Extrakte werden im Vakuum bis zur Trockne eingeengt und, eventuell nach Vorfraktionierung durch Flüssig-Flüssig-Extraktion, mit Hilfe eines oder mehrerer chromatographischen Verfahren aufgetrennt. Vorzugsweise wird hierfür die präparative HPLC an 'reversed-phase'-Material (RP₁₈) mit einem Wasser/Acetonitril- oder einem Wasser/Methanol-Gradienten verwendet. Als stationäre Phasen könnten auch Siliciumdioxid, Aluminiumoxid oder Cellulose Verwendung finden oder es könnte Flüssig-Flüssig-Chromatographie, z.B. HSCCC, eingesetzt werden. Es werden verschiedene Fraktionen gesammelt und durch HPLC oder Dünnschichtchromatographie auf ihren Gehalt an den erfindungsgemäßen Verbindungen untersucht. Nach Einengen der in Frage kommenden Fraktionen erhält man die Verbindungen in reiner Form.

### Beispiel 2: Beispiele für die erfindungsgemäßen Verbindungen. Isolierung von Sorbicillacton A

Der vorliegende Pilzstamm von Penicillium wurde am 02.05.2001 aus dem marinen Schwamm *Ircinia fasciculata* aus 17,5 m Wassertiefe in der Bucht von Fetovaia (42°43'24"N/10°09'31 "E) auf Elba, Italien, isoliert. Der Schwamm wurde sofort nach der Entnahme mit Hilfe einschlägiger marin-mykologischer Methoden auf seinen Pilzbesatz hin untersucht. Die vorliegende Isolation wurde durch Auslegen kleiner Gewebestückchen des sezierten Schwammes auf einer Nähragarplatte folgender Zusammensetzung (CYAS, nach Pitt 1973) gewonnen:
Czapek-Yeast Extract-Agar + Seawater:
   30 g Saccharose
   5 g Yeast Extract
   3 g NaNO₃
   1 g K₂HPO₄
   10 ml Mineral-Lösung:
      5 g KCI, 5g MgSO₄+7 H₂O, 0,1 g FeSO₄+7 H₂O / 100 ml H₂O
   1 ml Spurenmetall-Lösung:
      1 g ZnSO₄+7 H₂O, 0,5 g CuSO₄+5 H₂O / 100 ml H₂O
   Antibiotika
   1000 ml Meerwasser (30-33 PSU)

Durch mehrere Reinigungsstufen wurde die primäre Rohkultur zu einer axenischen Reinkultur weitergezüchtet. Die Stammkultur erfolgt auf Schrägagarröhrchen folgender Zusammensetzung (GPYNS, Schaumann 1974):
Glucose-Pepton-Hefeextrakt-Ammoniumnitrat-Meerwasser-Agar:
   1,0 g Glucose
   0,5 g Pepton
   0,1 g Hefeextrakt
   1,0 g Ammoniumnitrat
   15,0 g Agar
   1000 ml Meerwasser (30-33 PSU)
   P_{H} 7,2-7,4

Die Anzucht der Pilzkultur für die Gewinnung des neuen Naturstoffes Sorbicillacton A erfolgt in 1-L-Erlenmeyerkolben, die mit je 300 ml Nährlösung folgender Zusammensetzung (WS, nach Wickerham 1951) beschickt wurden:
Wickerham-Meerwasser-Medium:
   3,0 g Hefeextrakt
   3,0 g Malzextrakt
   5,0 g Pepton
   10,0 g Glucose
   1000 ml Meerwasser (30-33 PSU)
   P_{H} 7,2-7,4

Die Sterilisation der Nährlösung erfolgt durch Autoklavieren bei 121°C/1 bar, 15 Minuten. Als Inokulum für die experimentelle Pilzkultur dienen zehn Stück Mycelscheibchen (Durchmesser 5 mm) je Kolben. Diese werden aus einer 7 Tage alten Vorkultur auf WS-Agar mit Hilfe eines Korkbohrers ausgestanzt und in die Nährlösung übertragen. Die Inkubation der beimpften Kolben erfolgt über einen Zeitraum von 14 Tagen bei Raumtemperatur oder auch konstant 20°C in statischer Kultur im Dunkeln. Anschließend wird das gewachsene Mycel einschließlich der Kulturbrühe abgeerntet, mit 40 ml Ethylacetat pro 300 ml Kulturbrühe versetzt und bei -86°C tiefgefroren.

Bei drei 300-ml-Kulturansätzen wurde das Mycel vom Kulturmedium durch Filtration getrennt, zerkleinert und mit 250 ml eines Dichlormethan-Methanol-Gemisches (1:1) unter Rühren für 48 h extrahiert. Anschließend wurde das Mycel durch Zentrifugation abgetrennt und der Extrakt im Vakuum zur Trockne eingeengt. Das Kulturfiltrat wurde dreimal mit je 250 ml Essigester extrahiert, die Essigesterphasen vereinigt und ebenfalls im Vakuum zur Trockne eingeengt. Kulturfiltrat- und Mycelextrakt wurden zusammen in einem Gemisch aus 200 ml Methanol und 6 ml Wasser gelöst und mit 200 ml Petrolether extrahiert. Die Petroletherphase wurde verworfen, die Methanol-Wasser-Phase im Vakuum eingeengt und mittels HPLC-UV, -NMR und -MS untersucht. Durch Vergleich der so gewonnenen spektroskopischen Daten verschiedener Inhaltsstoffe mit Datenbanken konnten verschiedene bekannte Verbindungen, z.B. Meleagrin und Roquefortin C, identifiziert werden. Eine der Verbindungen wurde auf diese Weise als neuer, bisher unbekannter Naturstoff erkannt. Da zweidimensionale HPLC-NMR-Experimente am Extrakt, z.B. HPLC-WET-COSY und HPLC-WET-ROESY, zeigten, daß es sich hierbei um eine hochinteressante Struktur handelte, wurde diese Verbindung durch präparative HPLC isoliert:
Säule: Waters SymmetryPrep C18, 19 x 300 mm
Eluent: Acetonitril + 0.05% TFA, Wasser + 0.05% TFA
Gradient: von 10% Acetonitril auf 100% Acetonitril in 30 min
Fluß: 11 ml/min
Detektion: 254 nm

Sorbicillacton A eluierte zwischen 19 und 20 min. Die entsprechenden Fraktionen wurden gesammelt und im Vakuum bis zur Trockne eingeengt. Man erhielt 6 mg eines gelben, amorphen, in Methanol löslichen Feststoffs.

### Strukturaufklärung der Verbindung

Das gewonnene Sorbicillacton A hat folgende in Tabelle zusammengefaßten spektroskopische Eigenschaften:

**Tabelle 1:**

| NMR-Daten von Sorbicillacton A (ca. 6 mg in THF-d₈; 600MHz) | | | | | |
|---|---|---|---|---|---|
| Position | ¹³C [ppm] | ¹H [ppm] | COSY | HMBC | ROESY |
| 1 | 99.55 | | | | |
| 2 | 192.1 0 | | | | |
| 3 | 110.9 2 | | | | |
| 4 | 166.5 3 | | | | |
| 5 | 80.98 | | | | |
| 6 | 53.00 | 3.43, s | | 1,2,4,5,7,9, 11, 1' | 7, 11, 2' |
| 7 | ∼25.0 0 | 1.55, s | | 5,6 | 6 |
| 8 | 7.29 | 1.54, s | | 2,3,4 | |
| 9 | 59.98 | | | | |
| 10 | 172.9 8 | | | | |
| 11 | ~26.0 0 | 1.42, s | | 6, 9, 10 | NH, 6, 2' |
| 1' | 169.7 2 | | | | |
| 2' | 121.6 8 | 6.38, d | 3' (14,7 Hz) | 1',4' | 6,11 |
| 3' | 139.1 2 | 7.19, dd | 2', 4' (11 Hz) | 4', 5' | 5' |
| 4' | 131.9 5 | 6.28, ddd | 3', 5', 6' (1.3 Hz) | 6' | |
| 5' | 136.9 1 | 6.08, m | 4' (14.5 Hz), 6' (6.2 Hz) | 3', 6' | 3' |
| 6' | 18.54 | 1.83, dd | 4', 5' | 4', 5' | |
| 1" | 162.5 3 | | | | |
| 2" | 136.0 1 | 6.67, d | 3" (15.4 Hz) | 1 ", 3", 4" | NH |
| 3" | 131.2 2 | 6.49, d | 2" | 1 ", 2", 4" | |
| 4" | 166.3 1 | | | | |
| 1'-OH | | 16.60, s | | 1, 1', 2' | |
| NH | | 7.60, s | | 9, 10, 11, 1" | 11, 2" |

ESI-MS (in MeCN/H₂O): m/z 418 [M+H]⁺, 459 [M+MeCN+H]⁺
FAB-MS (in 3-Nitrobenzylalkohol) : m/z 418 [M+H]⁺
UV/VIS (in Acetonitril/Wasser + 0.05% TFA): λₘₐₓ [nm] 215, 271, 379

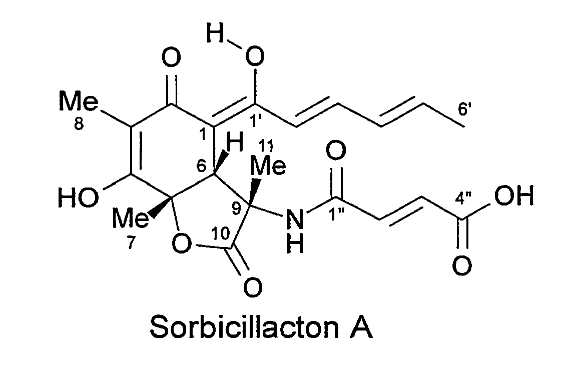

Die isolierte Verbindung, ein gelber, amorpher Feststoff, zeigt im ESI-MS ein [M+H]⁺⁻Signal bei m/z = 418, die daraus resultierende Molekülmasse von 417 wird auch durch die FAB-MS-Messung bestätigt.

Aus den NMR-Daten, vor allem den HMBC- und COSY-Korrelationen, lassen sich zwei Teilstrukturen des Moleküls ableiten. Ein Fumaratrest (5), bei welchem die E-Konfiguration der Doppelbindung durch die hohe Kopplungskonstante (15.4 Hz) belegt wird, und ein Sorbylrest (6). Wie dieser an den Rest des Moleküls gebunden ist, bleibt zunächst noch unklar, die für C-1' gemessene chemische Verschiebung (169.7 ppm) läßt sowohl die Möglichkeit eines Sorbinsäureesters als auch die eines C-C-gebundenen Sorbylrests in Enolform offen. Das Substitutionsmuster des zentralen 6-Rings **(7)** ist über HMBC-Wechselwirkungen der drei Methylgruppen C-7, C-8 und C-11 sowie von H-6 zugänglich.

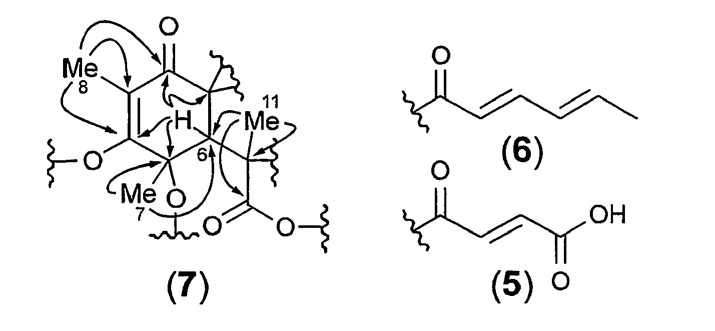

Die Durchführung der NMR-Messungen in THF-d₈ ermöglicht es, auch austauschbare Protonen und deren Wechselwirkungen zu beobachten. Hierbei erkennt man zwei zusätzliche scharfe Signale im ¹H-Spektrum, eine enolische Hydroxygruppe, die durch die Wasserstoffbrückenbindung zur β-ständigen Ketogruppe im Ring stark zu tiefem Feld verschoben ist (16.6 ppm), und ein amidisches Proton bei 7.6 ppm. Durch die HMBC-Korrelationen dieser Protonen können alle drei Teilstrukturen zu einer Gesamtstruktur **(8)** vereinigt werden.

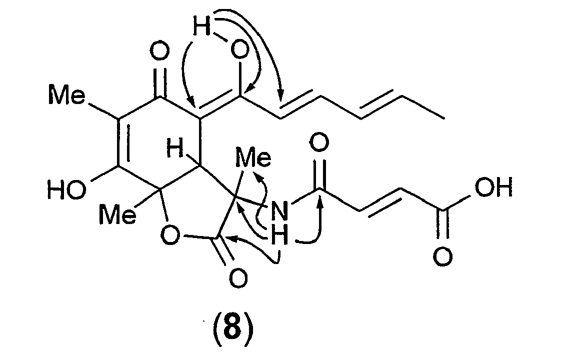

Zur Absicherung der Position des Lactonrings und der freien Säure- und Hydroxygruppen wurde der Naturstoff mit Diazomethan methyliert, wodurch ein dimethyliertes Derivat gebildet wurde, bei dem auch eine Cycloaddition von Diazomethan an die Doppelbindung des Fumaratrests stattgefunden hatte. NMR-Messungen an diesem Derivat führten zu der Struktur **(9)** [Sorbicillacton-A-Derivat 1 (SOA-D1)], womit für den Naturstoff die Struktur **(1)** feststeht. Die relative Konfiguration der drei Stereozentren läßt sich durch ROESY-Wechselwirkungen bestimmen: Die Korrelationen von H-6 zu den Methylgruppen 7 und 11 beweisen, daß beide cis-ständig zu H-6 sind.

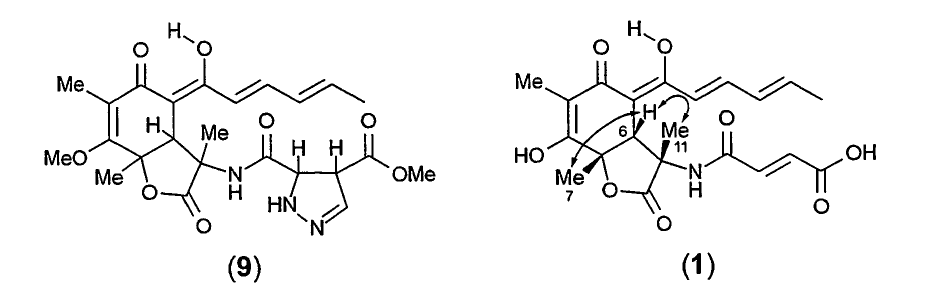

Auch die absolute Konfiguration des neuen Naturstoffes konnte aufgeklärt werden. Da es sich um einen völlig neuen Strukturtyp handelt, konnte hier nicht einfach eine Zuordnung durch Vergleich des CD-Spektrums mit dem einer strukturell verwandten konfigurativ bekannten Substanz vorgenommen werden. Die Zuordnung gelang aber durch Anwendung modemer quantenchemischer CD-Rechnungen, durch Simulation der für beide in Frage kommenden Enantiomeren zu erwartenden CD-Spektren und Vergleich dieser rechnerisch vorhergesagten Spektren mit dem tatsächlich für den Naturstoff experimentell gemessenen Spektrum. Durch diese Vorgehensweise wurde die vollständige absolute Stereostruktur von Sorbicillacton A wie in der Abbildung dargestellt etabliert.

### Beispiel 2: Derivatisierung von Sorbicillacton A zu Pyrazolderivaten

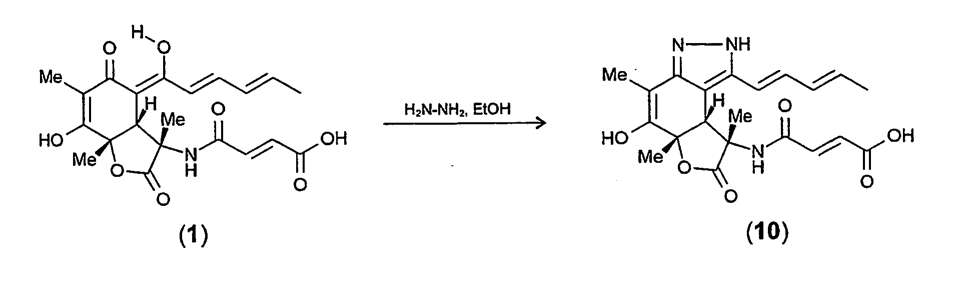

Zu einer Lösung von 100 mg Sorbicillacton A (1) in 2 ml Ethanol gibt man 10 mg Hydrazin. Nach 6 h Rühren bei Raumtemperatur verdampft man das Lösungsmittel. Die Aufreinigung des Rückstandes erfolgt mittels Säulenchromatographie an Kieselgel (Laufmittel: Dichlormethan-Methanol-Gemisch) und ergibt das gewünschte Pyrazolderivat **(10)**.

### Beispiel 3: Biologische Eigenschaften der Verbindungen

### a) Antitumorale Aktivität

Die antitumorale Aktivität von Sorbicillacton A und einem seiner Derivate (hier exemplarisch gezeigt an Derivat SOA-D1) wurde an einer Reihe von Tumor-transformierten Zellen, wie den L5178y Mäuselymphomzellsystem (ATCC CRL 1722) getestet. Wie beschrieben (Müller et al. (1979) Cancer Res. 39: 1102-1107) wurden die Zellen in RPMI-Medium, dem 10% fötales Kälberserum zugesetzt worden war, kultiviert. Als Inokulumskonzentration wurde 10.000 Zellen/ml gewählt. Zum Startzeitpunkt wurde die ausgewählte Substanz zugegeben und die Kultur 72 h inkubiert. Danach wurde die Anzahl der lebenden Zellen mittels des kolorimetrischen XTT-Ansatzes bestimmt und mit einem ELISA Reader ausgewertet (siehe: Scudiero DA, Shoemaker RH, Paull KD, Monks A, Tierney S, Nofziger TH, Currens MJ, Seniff D, Boyd MR (1988) Evaluation of a tetrazolium/formazan assay for cell growth and drug sensitivity in culture using human and other tumor cell lines. Cancer Res 48: 4827-4833; Daum T, Engels J, Mag M, Muth J, Lücking S, Schröder HC, Matthes E, Müller WEG (1992) Antisense oligonucleotides: inhibitors of splicing of mRNA of human immunodeficiency virus. Intervirology 33: 65-75). Die optische Dichte der Kontrollen wurde als 100% definiert.

| Verbindung | Konzentration der Verbindung (µg/ml) | Optische Dichte (595 nm) |
|---|---|---|
| Kontrolle | 0 | 0,38 |
| Sorbicillacton A | 0,1 | 0,24 |
| | 0,3 | 0,19 |
| | 1,0 | 0,07 |
| | 3,0 | 0,06 |
| SOA-D1 | 0,1 | 0,29 |
| | 0,3 | 0,22 |
| | 1,0 | 0,05 |
| | 3,0 | 0,03 |

*Ergebnis:* Es wird deutlich, daß bei den niedrigen Konzentration von > 0,1 µg/ml Sorbicillacton A und Derivate SOA-D1 die Zellproliferation nach 72 h entscheidend reduzieren. Die ED50-Konzentration (berechnet nach Sachs L. (1984) *Angewandte Statistik*. Springer, Berlin) lag bei den verwendeten L5178y Zellen bei 0,18 µg/ml.

Neben den Tumor-transformierten Zellen wurde der Einfluß der Verfahrensprodukte auch auf humane Vorhaut-Fibroblasten untersucht. Die Zellen und auch die Kultivierungsmethoden wurden früher beschrieben (siehe: Müller WEG, Maidhof A, Zahn RK, Schröder HC, Gasic MJ, Heidemann D, Bernd A, Kurelec B, Eich E, Seibert G (1985) Potent antileukemic activity of the novel cytostatic agent avarone and its analogues *in vitro* and *in vivo*. Cancer Res. 45: 4822-4827). Zellen zwischen der 6. und 9. Passage wurden für die Versuche eingesetzt. Die Kultivierung wurde in Kollagenbeschichteten Plastikflaschen durchgeführt. Die Zellzahl wurde mikroskopisch bestimmt. Die Untersuchungen ergeben, daß das Sorbicillacton A bei einer Konzentration von 30 µg/ml keinen Effekt auf die Zellteilung ausüben.

### b) Antivirale Aktivität

Eine ausführliche Angabe der Referenzen und der Durchführung der Methoden sind in früheren Arbeiten zusammengefaßt (Sarin PS, Sun D, Thornton A, Müller WEG (1987) Inhibition of replication of the etiologic agent of acquired immune deficiency syndrome (human T-lymphotropic retrovirus/lymphadenopathy-associated virus) by avarol and avarone. J Natl Cancer Inst 78: 663-666; Schröder HC, Sarin PS, Rottmann M, Wenger R, Maidhof A, Renneisen K, Müller WEG (1988) Differential modulation of host cell and HIV gene expression by combinations of avarol and AZT *in vitro.* Biochem Pharmacol 37: 3947-3952).

### Untersuchungsparameter: Zellwachstum

H9-Zellen sowie mit HTLV-IIIB (HIV-1) infizierte H9-Zellen wurden in einer Konzentration von 0,2 x 1 000 000 Zellen/ml Kulturmedium zum Beimpfen eines Kulturmediums verwendet. Nach 4-tägiger Inkubation betrug die Dichte der H9-Zellen 1,3 x 1 000 000 Zellen/ml, während die Dichte der mit HTLV-IIIB infizierten H9-Zellen lediglich 0,6 x 1 000 000 Zellen/ml war, diese beiden Werte bildeten die Kontrollwerte.

Dann wurden Proben von H9-HTLV-IIIB-Zellen (0,2 x 1 000 000 Zellen/ml) 4 Tage mit unterschiedlichen Konzentrationen von Sorbicillacton A behandelt. Es wurden folgende Ergebnisse erhalten:

| Verbindung | Konzentration der Verbindung (µg/ml) | Zellkonzentration x 1 000 000 / ml |
|---|---|---|
| Kontrolle | 0 | 0,62 |
| Sorbicillacton A | 0,1 | 0,68 |
| | 0,3 | 0,83 |
| | 1,0 | 1,39 |
| | 3,0 | 0,92 |

*Ergebnis:* Es ist ersichtlich, daß sowohl Sorbicillacton A in den Konzentrationen zwischen 0,3 und 3,0 µg/ml die Wachstumsrate von H9-HTLV-IIIB-Zellen auf Werte steigert, die im Bereich der Kontrolle, also H9-Zellen ohne HTLV-IIIB, liegen.

### Untersuchungsparameter: Produktion von Reverser Transkriptase

Bei diesem Ansatz wird geprüft, in welchem Umfange die Verfahrensprodukte die Produktion von HIV(HTLV-IIIB)-Viren in H9-Zellen hemmen. Als Parameter für die Menge an Viren wurde die in diesen Partikeln vorhandene Reverse Transkriptase gemessen.

Untersucht wurde, in welchem Umfange nach einer 4-tägigen Gabe von Sorbicillacton A oder dem Sorbicillacton-A-Derivat zu H9-HTLV-IIIB-Zellen die Produktion von HTLV-IIIB (HIV-1)-Viren reduziert wird. Als Maß für die Virusmenge im Kulturmedium wurde die Reverse Transkriptase ausgewählt, d.h. die Hemmung der Reverse-Transkriptase-Produktion zeigt die Hemmung der Virusproduktion an. Die Ergebnisse sind in nachfolgender Tabelle zusammengestellt:

| Verbindung | Konzentration der Verbindung (µg/ml) | Reverse Transkriptase Aktivität (100%) |
|---|---|---|
| Kontrolle | 0 | 100 |
| Sorbicillacton A | 0,1 | 83 |
| | 0,3 | 28 |
| | 1,0 | 22 |
| | 3,0 | 14 |

*Ergebnis:* Es wird ersichtlich, daß im Überstand der H9-HTLV-IIIB-Zellen, die nicht mit Sorbicillacton A behandelt wurden, eine beträchtliche Reverse-Transkriptase-Aktivität vorlag. Die Zugabe von Sorbicillacton A oder dem Sorbicillacton-A-Derivat führt zu einer dosisabhängigen Verringerung der Aktivität von Reverse Transkriptase im Überstand. Bereits bei einer Dosis von 0,1 µg/ml wurde eine beträchtliche Inhibierung beobachtet. Die erfindungsgemäß zur Anwendung kommenden Verbindungen sind deshalb in der Lage, die Virusreplikation beinahe vollständig zu inhibieren, in Konzentrationen, bei denen verschiedene *In-vitro*-Parameter, beispielsweise das Zellwachstum, praktisch nicht beeinflußt werden.

### Untersuchungsparameter: Expression der p24- und p15-Proteine

Es hat sich gezeigt, daß Sorbicillacton A und das Sorbicillacton-A-Derivat SOA-D1 eine stark inhibierende Wirkung auf die Expression von HIV p24 (Gag-Protein) und p15 (Gag-Protein) in infizierten H9-Zellen besitzen. Wurden die target-H9-Zellen mit dem HIV(HTLV-IIIB)-Isolat und ohne die zu testende Verbindung kultiviert, kam es, wie mittels indirekter Immunfluoreszenz-Assays festgestellt werden konnte, zur Expression des p24- und p15-Proteins. Nach Inkubation der H9-HTLV-IIIB-Zellen mit den zu testenden Verbindungen wurde jedoch ein beinahe vollständiger Schutzeffekt beobachtet. Die Expression der p24- und p15-Proteine wurde bis auf 24% reduziert. Es wurden folgende Ergebnisse erhalten:

| Verbindung | Konzentration der Verbindung (µg/ml) | Expression von p15 und p24 (in %) | |
|---|---|---|---|
| | | p15 | p24 |
| Kontrolle | 0 | 100 | 100 |
| Sorbicillacton A | 0,1 | 98 | 93 |
| | 0,3 | 65 | 42 |
| | 1,0 | 31 | 28 |
| | 3,0 | 47 | 24 |

*Ergebnis:* Es ist ersichtlich, daß Sorbicillacton A und das Sorbicillacton-A-Derivat (SOA-D1) eine signifikante Reduktion der Expression der HTLV-IIIB (HIV-1)-Proteine verursachen.

### Beispiel 4: Effekt von Sorbicillacton A in vivo

Für diese Untersuchungen wurden männliche (outbred) NMRI-Mäuse (32-36g; Alter: 8-9 Monate) eingesetzt. Die Testsubstanz Sorbicillacton A wurde in Methylzellulose gelöst und den Tieren i.p. injiziert. Fünf Tage lang wurde eine Dosis von 20 mg/kg (pro Tag) den Tieren verabreicht. Nach der Behandlung wurde das Gewicht der Tiere bestimmt. Während dieser Zeit veränderte sich das Gewicht der Sorbicillacton-A-behandelten Tiere (33±4 g) von denen der Kontrollen [nicht mit Sorbicillacton A behandelt] (35±4 g) nicht signifikant. Keines der Versuchstiere starb.

*Ergebnis:* Aus diesen Daten wird geschlossen, daß die subakute Toxizität von Sorbicillacton A bei einer fünftägigen i.p.-Behandlung >>20 mg/kg beträgt.

### Beschreibung der Synthese von Sorbicillacton A und deren Derivaten

Sorbicillacton A und eine ganze Reihe struktureller Analoga lassen sich durch biomimetische Synthese, ausgehend von Sorbicillin und verwandten Verbindungen, durch oxidative Dearomatisierung und anschließende Addition von Alanin (im Falle von Sorbicillacton A) oder anderen Aminosäuren und deren Analoga und anschließende Anknüpfung von Fumarsäure (im Falle von Sorbicillacton A) oder analogen Acylresten in wenigen Schritten herstellen.

## Patentansprüche

1. Verbindung der allgemeinen Formel (2): 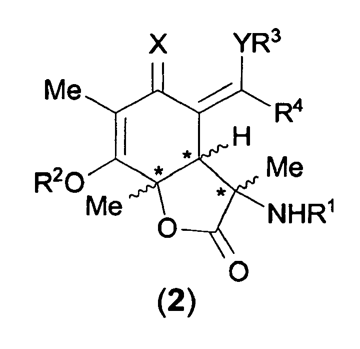 worin
R¹ ist ausgewählt aus: -H, (C₁-C₁₀)-Alkyl, wobei Alkyl gerade oder verzweigt ist, (C₃₋C₁₀)-Alkenyl oder eine Acylgruppe (z.B. Formyl, Acetyl, Trichloracetyl, Fumaryl, Maleyl, Succinyl usw.), wobei eventuelle freie -COOH-Gruppen an dieser Acylgruppe auch in Form von Estern (z.B. eines Methylesters, -COOMe) vorliegen können oder R¹ gegebenenfalls auch einer der beiden heterocyclischen Acylsubstituenten (3) oder **(4)** sein kann 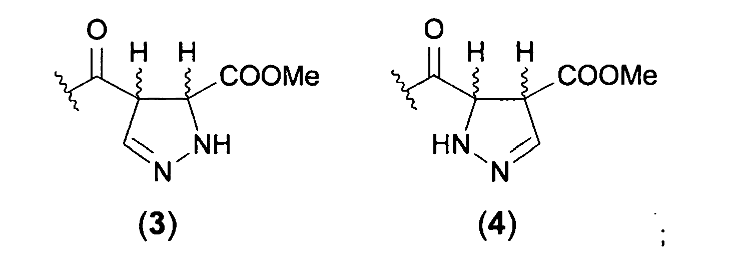
R² ausgewählt ist aus: -H, (C₁-C₁₀)-Alkyl, wobei Alkyl gerade oder verzweigt ist oder einer Acylgruppe (z.B. Formyl, Acetyl usw.);
R³ ausgewählt ist aus: -H, (C₁-C₁₀)-Alkyl, wobei Alkyl gerade oder verzweigt ist oder eine Acylgruppe (z.B. Formyl, Acetyl usw.);
R⁴ ausgewählt ist aus: (C₁-C₁₀)-Alkyl, wobei Alkyl gerade oder verzweigt ist oder (C₃₋C₁₀)-Alkenyl, wobei der Alkenylrest entweder eine oder mehrere Doppelbindungen enthalten kann;
X ausgewählt ist aus O, S, NOH oder NOR⁵, wobei R⁵ ein geradekettiges oder verzweigtes (C₁-C₆)-Alkyl ist;
Y entweder O ist oder Y und X sind zwei miteinander verbundene N-Atome, die somit einen Pyrazolring bilden.
und wobei die Verbindung als (*R,R,R*)-, (*R,R,S*)-, (*R,S,R*)-, (*R,S,S*)-, (*S,R,R*)-, (*S,R,S*)-, (*S,S,R*)- und (*S,S,S*)-Stereoisomer vorliegen kann und pharmazeutisch verträgliche Salze oder Solvate von (2).

2. Verbindung nach Anspruch 1 mit der Formel (1): 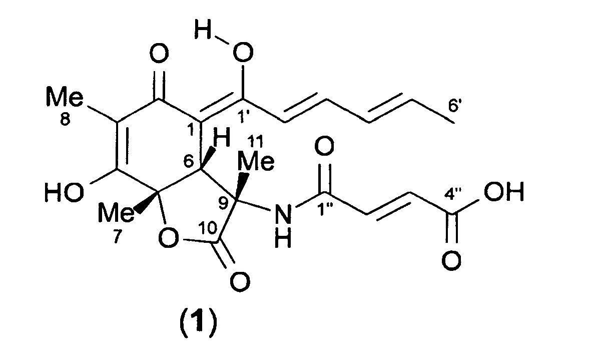 (Sorbicillacton A) oder Derivate davon, deren Diastereomere, sowie die entsprechenden Enantiomere und pharmazeutisch verträgliche Salze oder Solvate dieser Verbindung.

3. Verfahren zur Herstellung einer Verbindung nach Anspruch 1 oder 2, umfassend Züchten eines Pilzes der Gattung *Penicillium,* insbesondere *Penicillium chrysogenum,* auf an sich bekannte Weise und Isolieren mindestens einer erfindungsgemäßen Verbindung aus dem Kulturmedium und/oder der Pilzbiomasse.

4. Verfahren nach Anspruch 3, **dadurch gekennzeichnet, daß** das Anzüchten des Pilzes in einem marinen Organismus, insbesondere dem marinen Schwamm *Ircinia fasciculata* (Porifera), erfolgt.

5. Verfahren nach Anspruch 3 oder 4, weiterhin umfassend eine anschließende synthetische Derivatisierung der isolierten Verbindung.

6. Verfahren zur biomimetischen Synthese einer Verbindung nach Anspruch 1 oder 2, umfassend
a) zur Verfügung stellen von Sorbicillin und/der Derivaten davon,
b) oxidative Dearomatisierung und anschließende Addition von Alanin oder anderen Aminosäuren und deren Analoga, und
c) anschließende Anknüfung von Fumarsäure oder analogen Acylresten.

7. Verbindung nach Anspruch 1 oder 2 zur Verwendung zur Behandlung von Erkrankungen.

8. Pharmazeutische Zusammensetzung, umfassend eine Verbindung nach Anspruch 1 oder 2 zusammen mit geeigneten Zusatz- oder Hilfsstoffen.

9. Pharmazeutische Zusammensetzung nach Anspruch 8, **dadurch gekennzeichnet, daß** die Verbindung in Form einer Depotsubstanz oder als Vorläufer zusammen mit einer geeigneten, pharmazeutisch verträglichen Verdünnungslösung oder Trägersubstanz vorliegt.

10. Pharmazeutische Zusammensetzung nach Anspruch 8 oder 9, **dadurch gekennzeichnet, daß** die Verbindung in einer Menge von 20 µg vorliegt.

11. Pharmazeutische Zusammensetzung nach Anspruch 8 oder 9, **dadurch gekennzeichnet, daß** die Verbindung in einer Menge vorliegt, daß ein Konzentrationsbereich zwischen 0,3 und 3,0 µg/ml bei der Behandlung in vivo vorliegt.

12. Pharmazeutische Zusammensetzung nach einem der Ansprüche 8 bis 11, **dadurch gekennzeichnet, daß** sie weitere Chemotherapeutika enthält.

13. Pharmazeutische Zusammensetzung nach einem der Ansprüche 8 bis 12 in Form von Tabletten, Dragees, Kapseln, Tropflösungen, Suppositorien, Injektions- oder Infusionszubereitungen zur peroralen, rektalen oder parenteralen Verwendung.

14. Verwendung einer Verbindung nach Anspruch 1 oder 2 zur Herstellung eines Medikaments zur Behandlung von Tumor- und/oder Viruserkrankungen und/oder zur Behandlung von Entzündungszuständen.

15. Verwendung nach Anspruch 14 in Form einer Depotsubstanz oder als Vorläufer zusammen mit einer geeigneten, pharmazeutisch verträglichen Verdünnungslösung oder Trägersubstanz.

16. Verwendung nach Anspruch 14 oder 15 zur Behandlung von HIV-1 in einem Konzentrationsbereich zwischen 0,3 und 3,0 µg/ml.

17. Verwendung nach Anspruch 14 oder 15 zur Behandlung von Entzündungen in einer Konzentration von 2 µg/ml.

18. Verwendung nach Anspruch 14 oder 15 zur Behandlung der Ödembildung in einer Menge von 20 µg.

## Claims

1. Compound of the general formula (2): 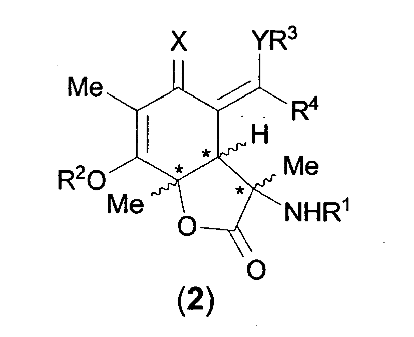 wherein
R¹ is selected from: -H, (C₁-C₁₀)-alkyl, wherein alkyl is straight or branched, (C₃-C₁₀)-alkenyl or an acyl group (e.g. formyl, acetyl, trichloroacetyl, fumaryl, maleyl, succinyl, etc.), wherein eventual free -COOH-groups at this acyl group can also be present in the form of esters (e.g. a methyl ester, -COOMe); or R¹, optionally, can also constitute one of both heterocyclic acyl substituents **(3)** or **(4)** 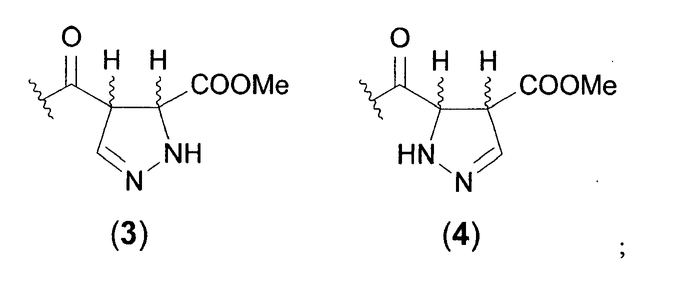
R² is selected from: -H, (C₁-C₁₀)-alkyl, wherein alkyl is straight or branched, or an acyl group (e.g. formyl, acetyl etc.);
R³ is selected from: -H, (C₁-C₁₀)-alkyl; wherein alkyl is straight or branched, or an acyl group (e.g. formyl, acetyl etc.);
R⁴ is selected from: (C₁-C₁₀)-alkyl, wherein alkyl is straight or branched or (C₃-C₁₀)-alkenyl, wherein the alkenyl residue can either contain one or several double bonds;
X is selected from O, S, NOH or NOR⁵, wherein R⁵ is a straight chain or branched (C₁-C₆)-alkyl;
Y is either O or Y, and X is two connected N-atoms, thus forming a pyrazole ring,
and wherein the compound can be present as (*R*,*R*,*R*)-, (*R*,*R*,*S*)-, (*R*,*S*,*R*)-, (*R*,*S*,*S*)-, (*S*,*R*,*R*)-, *(S,R,S)-,* (*S*,*S*,*R*)-, and (*S*,*S*,*S*)-stereoisomers, and pharmaceutically acceptable salts or solvates of (2).

2. Compound according to claim 1 with the formula (1): 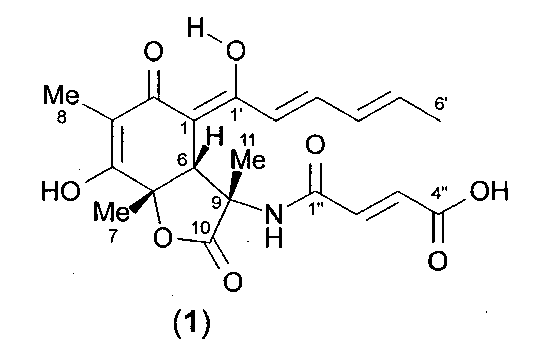 (sorbicillacton A) or derivatives thereof, their diastereomers, as well as the respective enantiomers, and pharmaceutically acceptable salts or solvates of this compound.

3. Method for producing a compound according to claim 1 or 2, comprising culturing of a fungus of the genus *Penicillium,* in particular *Penicillium chrysogenum,* in a manner known as such, and isolating of at least one compound according to the invention from the culture medium, and/or the fungal biomass.

4. Method according to claim 3, **characterized in that** culturing of the fungus occurs in a marine organism, in particular the marine sponge *Ircinia fasciculata* (*porifera*).

5. Method according to claim 3 or 4, further comprising a subsequent synthetic derivatization of the isolated compound.

6. Method for the biomimetic synthesis of a compound according to claim 1 or 2, comprising
a) providing of sorbicillin and/or derivates thereof,
b) oxidative de-aromatisation, and subsequent addition of alanin or other amino acids and their analogs, and
c) subsequent attachment of fumaric acid or analogous acyl residues.

7. Compound according to claim 1 or 2 for use for the treatment of diseases.

8. Pharmaceutical composition, comprising a compound according to claim 1 or 2 together with suitable additives or excipients.

9. Pharmaceutical composition according to claim 8, **characterized in that** the compound is present in form of a depot substance or as precursor, together with a suitable pharmaceutically acceptable diluents or carrier substances.

10. Pharmaceutical composition according to claim 8 or 9, **characterized in that** the compound is present in an amount of 20 µg.

11. Pharmaceutical composition according to claim 8 or 9, **characterized in that** the compound is present in an amount, so that a range of concentrations between 0.3 and 3.0 µg/ml is present during a treatment in vivo.

12. Pharmaceutical composition according to any of claims 8 to 11, **characterized in that** it contains additional chemotherapeutics.

13. Pharmaceutical composition according to any of claims 8 to 12 in the form of tablets, dragees, capsules, drop solutions, suppositories, preparations for injection or infusion for a peroral, rectal or parenteral use.

14. Use of a compound according to claim 1 or 2 for producing a medicament for the treatment of tumorous and/or viral diseases und/or for the treatment of inflammatory conditions.

15. Use according to claim 14 in form of a depot substance or as precursor together with a suitable pharmaceutically acceptable diluent or carrier substance.

16. Use according to claim 14 or 15 for the treatment of HIV-1 in a range of concentrations between 0.3 and 3.0 µg/ml.

17. Use according to claim 14 or 15 for the treatment of inflammations in a concentrations of 2 µg/ml.

18. Use according to claim 14 or 15 for the treatment of edema formation in an amount of 20 µg.

## Revendications

1. Composé de formule générale (2) : 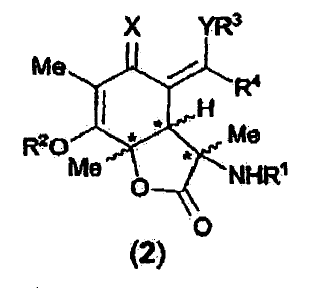 Où
R¹ est choisi parmi : -H, -alkyle en C₁-C₁₀, le groupement alkyle étant linéaire ou ramifié, -alcényle en C₃-C₁₀ ou un groupement acyle (par exemple formyle, acétyle, trichloroacétyle, fumaryle, maléyle, succinyle, etc.), d'éventuels groupes -COOH libres sur ces groupements acyles pouvant également être présents sous forme d'esters (par exemple d'un ester de méthyle, - COOMe) ou bien R¹ pouvant également être le cas échéant l'un des deux substituants hétérocycliques acylés (3) ou (4) 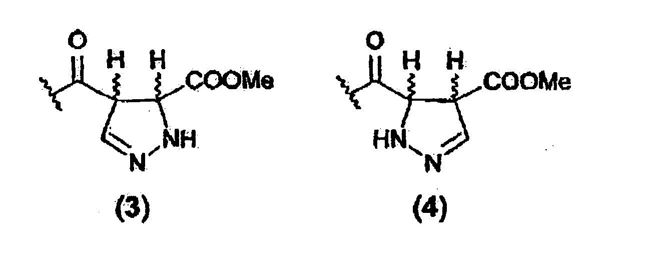
R² est choisi parmi : -H, -alkyle en C₁-C₁₀, le groupement alkyle étant linéaire ou ramifié ou un groupement acyle (par exemple formyle, acétyle etc.) ;
R³ est choisi parmi : -H, -alkyle en C₁-C₁₀, le groupement alkyle étant linéaire ou ramifié ou un groupement acyle (par exemple formyle, acétyle etc.) ;
R⁴ est choisi parmi : -alkyle en C₁-C₁₀, le groupement alkyle étant linéaire ou ramifié, ou - alcényle en C₃-C₁₀, le radical alcényle pouvant comprendre une ou plusieurs doubles liaisons ;
X est choisi parmi O, S, NOH ou NOR⁵, R⁶ étant un alkyle en C₁-C₆ linéaire ou ramifié ;
soit Y représente O, soit Y et X représentent deux atomes de N liés entre eux qui forment ainsi un cycle pyrazole,
et moyennant quoi le composé peut être présent en tant que stéréoisomère (R, R, R), (R, R, S), (R, S, R), (R,S,S), (S,R,R), (S,R,S), (S,S,R) et (S,S,S) et des sels ou solvats pharmaceutiquement acceptables de (2).

2. Composé selon la revendication 1 ayant la formule (1) : 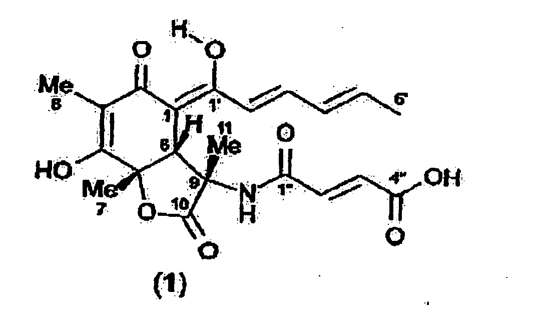 (sorbicillactone A) ou des dérivés de celui-ci, ses diastéréomères ainsi que les énantiomères correspondants et les sels ou solvats pharmaceutiquement acceptables de ce composé.

3. Procédé de fabrication d'un composé selon la revendication 1 ou 2, comprenant la culture d'un champignon du genre *penicillium,* en particulier *penicillium chrysogenum*, de manière connue en elle-même et l'isolation d'au moins un composé selon l'invention à partir du milieu de culture et/ou de la biomasse du champignon.

4. Procédé selon la revendication 3, **caractérisé en ce que** la culture du champignon se produit dans un organisme marin, en particulier l'éponge marine *ircinia fasciculata (porifera)*.

5. Procédé selon la revendication 3 ou 4 comprenant en outre une dérivatisation synthétique ultérieure du composé isolé.

6. Procédé de synthèse biomimétique d'un composé selon la revendication 1 ou 2, comprenant
a) la mise à disposition de sorbicilline et/ou de dérivés de celle-ci,
b) la désaromatisation oxydative et l'addition ultérieure d'alanine ou d'autres acides aminés et les analogues de ceux-ci, et
c) l'attachement suivant d'acide fumarique ou de radicaux acyles analogues.

7. Composé selon la revendication 1 ou 2 pour l'utilisation dans le traitement de maladies.

8. Composé pharmaceutique comprenant un composé selon la revendication 1 ou 2 conjointement avec des additifs ou des adjuvants appropriés.

9. Composé pharmaceutique selon la revendication 8, **caractérisé en ce que** le composé est présent sous forme d'une substance à libération contrôlée ou en tant que précurseur conjointement avec une solution de dilution ou une substance support, pharmaceutiquement acceptable et appropriée.

10. Composé pharmaceutique selon la revendication 8 ou 9, **caractérisé en ce que** le composé est présent en une quantité de 20 µg.

11. Composé pharmaceutique selon la revendication 8 ou 9, **caractérisé en ce que** le composé est présent dans une quantité telle qu'un domaine de concentration entre 0,3 et 3,0 µg/ml est présent in vivo pendant le traitement.

12. Composé pharmaceutique selon l'une quelconque des revendications 8 à 11, **caractérisé en ce qu'**il contient d'autres substances chimiothérapiques.

13. Composé pharmaceutique selon l'une quelconque des revendications 8 à 12, sous forme de comprimés, dragées, capsules, solutions au goutte à goutte, suppositoires, préparations à injection ou à infusion pour l'utilisation pérorale, rectale ou parentérale.

14. Utilisation d'un composé selon la revendication 1 ou 2 pour la fabrication d'un médicament pour traiter des maladies tumorales et/ou virales et/ou pour traiter des états inflammatoires.

15. Utilisation selon la revendication 14 sous forme d'une substance à libération contrôlée ou en tant que précurseur conjointement avec une solution de dilution ou une substance support, pharmaceutiquement acceptable et appropriée.

16. Utilisation selon la revendication 14 ou 15 pour le traitement du VIH-1 dans un domaine de concentration entre 0,3 et 3,0 µg/ml.

17. Utilisation selon la revendication 14 ou 15 pour le traitement d'inflammations en une concentration de 2 µg/ml.

18. Utilisation selon la revendication 14 ou 15 pour le traitement de la formation d'oedème en une quantité de 20 µg.
